(19)

**Europäisches Patentamt**
**European Patent Office**
**Office européen des brevets**

(11) **EP 3 951 681 A1**

(12) **EUROPÄISCHE PATENTANMELDUNG**

(43) Veröffentlichungstag:
**09.02.2022 Patentblatt 2022/06**

(21) Anmeldenummer: **20189656.0**

(22) Anmeldetag: **05.08.2020**

(51) Internationale Patentklassifikation (IPC):
**G06Q 10/08** *(2012.01)* **G16Y 20/10** *(2020.01)*

(52) Gemeinsame Patentklassifikation (CPC):
**G16Y 20/10; G06Q 10/0832**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder:
• **Tsenso GmbH**
**70176 Stuttgart (DE)**

• **Fleck, Christian**
**79423 Heitersheim (DE)**

(72) Erfinder:
• **Fleck, Christian**
**79423 Heitersheim (DE)**
• **Brunner, Matthias**
**70195 Stuttgart (DE)**

(74) Vertreter: **Patentanwaltskanzlei Cartagena Partnerschaftsgesellschaft Klement, Eberle mbB**
**Urbanstraße 53**
**70182 Stuttgart (DE)**

(54) **VERFAHREN ZUR ERMITTLUNG VON EIGENSCHAFTEN VON LEBENSMITTELN**

(57) Zur Ermittlung von Eigenschaften von Lebensmitteln wird ein Verfahren vorgeschlagen, bei dem zu einem Lebensmittel eine digitale Zwillingsinstanz (300) Verwendung findet, um aktuelle oder zukünftige Eigenschaften des Lebensmittels abfragen zu können.

Hierfür wird ausgehend von einer digitalen Zwillingsvorlage (200) eine digitale Zwillingsinstanz (300) als Repräsentation des Lebensmittels erzeugt. Der digitalen Zwillingsinstanz (300) ist für mindestens eine erste Zielvariable (304), die der Beschreibung einer Eigenschaft des Lebensmittels dient, ein mathematisches Modell (306), welches über mindestens einen Modellparameter und mindestens über einen Umgebungsparameter (310) verfügt, zugeordnet. Weiterhin ist der digitalen Zwillingsinstanz (300) eine Wahrscheinlichkeitsverteilung (308) in Hinblick auf den mindestens einen Modellparameter des mathematischen Modells (306) der ersten Zielvariablen zugeordnet.

Im Zuge der Handhabung des Lebensmittels bis zum Erreichen einer Verkaufsstätte (16) und/oder an der Verkaufsstätte (16) erfolgt mindestens eine Messung des mindestens einen Umgebungsparameters (310), wobei die hierbei ermittelten Werte des Umgebungsparameters (310) in der Zwillingsinstanz (300) zugeordneter Weise abgespeichert werden. Unter Verwendung des mathematischen Modells (306) der ersten Zielvariable (304), der Wahrscheinlichkeitsverteilung (308) des mindestens einen Modellparameters und der ermittelten Werte des mindestens einen Umgebungsparameters (310) kann dann für den aktuellen Zeitpunkt oder einen zukünftigen Zeitpunkt eine Wahrscheinlichkeitsverteilung (320) in Hinblick auf die mindestens eine Zielvariable (304) ermittelt werden.

Fig. 2A

**Beschreibung**

ANWENDUNGSGEBIET UND STAND DER TECHNIK

Die Erfindung betrifft ein Verfahren zur Ermittlung von Eigenschaften von Lebensmitteln.

Lebensmittel sind üblicherweise nur zeitlich begrenzt haltbar. Ausgehend von Zeitpunkt Ihrer Herstellung verschlechtert sich ihre Qualität, wobei diese Verschlechterung sowohl gesundheitlich nachrangige Eigenschaften wie Veränderungen des Aromas, der Farbe oder der Konsistenz betreffen als auch gesundheitlich beim Verzehr relevante Eigenschaften wie Bakterienbefall und -vermehrung sowie Bildung von Giftstoffen betreffen können.

Um dem Verbraucher mitzuteilen, wie lange ein Lebensmittel ohne wesentliche Geschmacks- und Qualitätseinbußen sowie ohne gesundheitliches Risiko zu konsumieren ist, ist auf vielen Lebensmitteln ein Mindesthaltbarkeitsdatum angegeben, welches üblicherweise bei der Produktion des Lebensmittels auf dessen Verpackung aufgebracht wird. Bei leicht verderblichen Lebensmitteln mit besonders großer Gefahr für die Gesundheit bei zu später Verwendung, wird häufig statt eines Mindesthaltbarkeitsdatum ein Verbrauchsdatum angegeben, nach dem ein Verzehr auf jeden Fall unterbleiben sollte. Die genannte Gefahr ergibt sich primär aus so genannten pathogenen Keimen, die Krankheiten auslösen, wenn die Konzentration dieser Keime aufgrund erfolgter Vermehrung im Lebensmittel zu groß ist.

Der Zeitpunkt der Aufbringung des jeweiligen Datums bei der Produktion oder Verpackung des Lebensmittels gestattet es nicht, die anschließende Handhabung des Lebensmittels hierbei zu berücksichtigen. Dies betrifft sowohl die Handhabung des Lebensmittels zwischen der Produktions- oder Verpackungsstätte und der Verkaufsstätte, beispielsweise einem Supermarkt, als auch die Handhabung des Lebensmittels nach Verkauf in der Verkaufsstätte, also beispielsweise beim Transport des Lebensmittels vom Supermarkt in die Wohnung des Käufers sowie bei der Lagerung des Lebensmittels beim Käufer, insbesondere in dessen Kühlschrank.

Aus der DE 60217329 T2 ist es bekannt, die Qualität von Lebensmitteln abzuschätzen, indem ihre Temperatur in der Lieferkette erfasst wird und auf Basis des Temperaturverlaufs und einer initialen Keimzahl eine aktuelle Keimzahl ermittelt wird. In Abhängigkeit des Temperaturverlaufs werden weiterhin Warnmitteilungen erzeugt.

In der Praxis ist das Errechnen der aktuellen Keimzahl auf Basis einer initialen Keimzahl jedoch schwierig, da eine konkrete initiale Keimzahl nur schwer festzulegen ist und da das Verhalten der Keime bei unterschiedlichen Temperaturen nicht in dem Maße prognostizierbar ist, wie es für die Realisierung der Vorschläge der DE 60217329 T2 erforderlich wäre.

AUFGABE UND LÖSUNG

Aufgabe der Erfindung ist es, ein Verfahren zur Verfügungzu stellen, welches es gestattet Eigenschaften von Lebensmitteln zu ermitteln.

Das erfindungsgemäße Verfahren sieht hierzu vor, dass zu einem Lebensmittel ausgehend von einer digitalen Zwillingsvorlage eine digitale Zwillingsinstanz als Repräsentation des Lebensmittels erzeugt wird.

Dieser digitalen Zwillingsinstanz sind dabei mindestens die folgenden Informationen zugeordnet:
Für mindestens eine erste Zielvariable, die der Beschreibung einer Eigenschaft des Lebensmittels dient, verfügt die Zwillingsinstanz über ein mathematisches Modell, welches seinerseits über mindestens einen Modellparameter und mindestens über einen Umgebungsparameter verfügt.

Weiterhin verfügt die digitale Zwillingsinstanz über mindestens eine Wahrscheinlichkeitsverteilung in Hinblick auf den mindestens einen Modellparameterdes mathematischen Modells der ersten Zielvariablen.

Unter einer Wahrscheinlichkeitsverteilungwird im Sinne dieser Erfindung eine Funktion verstanden, die jedem Wert des zugehörigen Modellparameters eine Wahrscheinlichkeit seines Eintretens zuordnet, wobei für mindestens zwei mögliche Werte des zugehörigen Modellparameters unterschiedliche Wahrscheinlichkeiten gegeben sind.

Im Zuge der Handhabung des Lebensmittels bis zum Erreichen einer Verkaufsstätte und/oder an der Verkaufsstätte erfolgt mindestens eine Messung des mindestens einen Umgebungsparameters, wobei die hierbei ermittelten Werte des Umgebungsparameters in der Zwillingsinstanz zugeordneter Weise abgespeichert werden. Auf diese Weise wird die Zwillingsinstanz mit Daten zur Umgebung ergänzt, denen das reale Lebensmittel ausgesetzt ist.

Zur Ermittlung von Eigenschaften des Lebensmittels wird unter Verwendung des mathematischen Modells der Zielvariablen, der Wahrscheinlichkeitsverteilung des mindestens einen Modellparameters und der ermittelten Werte des mindestens einen Umgebungsparameters für den aktuellen Zeitpunkt oder einen zukünftigen Zeitpunkt eine Wahrscheinlichkeitsverteilung in Hinblick auf die mindestens eine Zielvariable ermittelt.

Das erfindungsgemäße Verfahren sieht also vor, dass durch die digitale Zwillingsinstanz, die eine Struktur digitaler Daten ist, die einem Lebensmittel bzw. dessen Charge zugeordnet ist, ein Werkzeug zur Verfügung gestellt wird, welches jederzeit praktisch verwendbare Informationen dazu bereitstellen kann, ob und ggf. wie lange das Lebensmittel in Hinblick auf eine Zielvariable eine bestimmte Qualität aufweist, üblicherweise eine gute Qualität, also nicht gesundheitlich bedenklich und/oder nicht ästhetisch oder geschmacklich beanstandenswert ist.

Dabei versucht das erfindungsgemäße Verfahren gezielt nicht, eine absolute Aussage über die Qualität zu treffen.

Eine absolute Aussage, wie beispielsweise "Die Listerienkonzentration des Lebensmittels liegt bei X" oder "Die Listerienkonzentration des Lebensmittels liegt oberhalb von Y" ist in der Praxis nicht seriös für alle Einzellebensmittel einer Charge zu treffen, insbesondere dann nicht, wenn bereits das Zustandekommen einer solchen Aussage auf einer mathematischen Betrachtung der zeitlichen Veränderung von Zielvariablen aus Basis von Modellparamatern erfolgt, die einfache statistische Mittelwerte betreffen.

Das erfindungsgemäße Verfahren hingegen arbeitet in Hinblick auf mindestens einen Modellparameter des mathematischen Modells der mindestens einen Zielvariablen mit der genannten Wahrscheinlichkeitsverteilung und errechnet demzufolge auch die Zielvariable als Wahrscheinlichkeitsverteilung.

Neben der Tatsache, dass die Errechnung der Zielvariable als Wahrscheinlichkeitsverteilung die Wahrheit über das betroffene Lebensmittel besser wiederspiegelt als eine Errechnung eines absoluten Wertes, der in der Regel inhaltlich falsch sein dürfte, gestattet die Wahrscheinlichkeitsverteilung der Zielvariable angepasste Aussagen zu spezifischeren Fragestellungen die Qualität betreffend.

So ist es beispielsweise möglich, aus der Zielvariablen nicht nur abzuleiten, dass das Lebensmittel im statistischen Mittel von guter oder hervorragender Qualität ist, sondern auch mit welcher Wahrscheinlich dies nicht der Fall ist und das Lebensmittel möglicherweise nicht mehr genießbar ist. Derartiges gestattet auch differenziertere Antworten auf die Frage der Verwendbarkeit der Lebensmittel. So mag aufgrund der stärkeren Konstitution von Erwachsenen gegenüber Kleinkindern ein Lebensmittel, welches nur im statistischen Mittel von guter Qualität ist, ungefährdet von Erwachsenen konsumiert werden, während Kleinkinder aufgrund der doch zu hohen Wahrscheinlichkeit schlechterer Qualität das Lebensmittel nicht konsumieren sollten.

Dass das erfindungsgemäße Verfahren die Zielvariable als Wahrscheinlichkeitsverteilung errechnet, steht einer absoluten Aussage nicht entgegen. Insbesondere für die Informationsaufbereitung für Endkunden, beispielsweise auf seinem Mobiltelefon nach dem Einscannen eines QR-Codes auf dem Lebensmittel, ist eine Wahrscheinlichkeitsverteilung meist nicht gut geeignet, da hier eine eher klare Aussage gewünscht ist. Hier böte sich daher beispielsweise eher an, zur betreffenden Zielvariable die Qualität anzugeben, die gemäß der Wahrscheinlichkeitsverteilung für die besten 99,99% der Lebensmittel mindestens gegeben ist. In der Praxis ist es für den Endkunden in der Regel auch nicht hilfreich, eine Vielzahl einzelner Zielvariablen zu erfahren, sondern diese sollten in eine gemeinsame Bewertung münden wie beispielsweise "Bis 15.7. bei Lagerung bei 6°C ästhetisch und geschmacklich einwandfrei, bis 30.7. bei Lagerung bei 6°C gesundheitlich für Erwachsene unbedenklich".

Vorzugsweise ist zum Zwecke der Ausgabe einer einfacher verständlichen Ausgabe ausgehend von der ermittelten Wahrscheinlichkeitsverteilung der Zielvariable vorgesehen, dass unter Verwendung der Wahrscheinlichkeitsverteilung in Hinblick auf die mindestens eine Zielvariable durch Aufsummieren der Fläche unter der Wahrscheinlichkeitsverteilung ermittelt wird, mit welcher kumulierten Wahrscheinlichkeit die Zielvariable des Lebensmittels unterhalb bzw. oberhalb eines vorgegebenen Grenzwertes für die Zielvariable liegt. Alternativ kann unter Verwendung der Wahrscheinlichkeitsverteilung in Hinblick auf die mindestens eine Zielvariable durch Aufsummieren der Fläche unter der Wahrscheinlichkeitsverteilung ermittelt werden, welcher Wert der Zielvariable statistisch bei einem vorgegebenen Anteil des Lebensmittels unterschritten bzw. überschritten ist.

Vorzugsweise weist die digitale Zwillingsinstanz ein mathematisches Modell sowie mindestens einen dem mathematischen Modell zugeordneten Modellparameter, der in Form einer Wahrscheinlichkeitsverteilung vorliegt, für mindestens ein mikrobiologische Zielvariablen auf. Zu mikrobiologischen Zielvariablen werden insbesondere gezählt: Konzentration in Hinblick auf beliebige Keime, Konzentration von Listerien, Konzentration von Lactobacillales, Konzentration von Cronobacter, Konzentration von Bacillus cereus, Konzentration von Campylobacter, Konzentration von Salmonellen, Konzentration von Shigellen, Konzentration von Staphylococcus aureus, Konzentration von Pseudomonas spp. Konzentration von Schimmelpilz und Konzentration von Aspergillus spp..

Es handelt sich hierbei um Zielvariablen, deren Auswertung für die Bewertung, ob das Lebensmittel noch gesundheitlich unbedenklich verzehrt werden kann, von Relevanz sind.

Alternativ oder zusätzlich kann die Zwillingsinstanz jedoch auch mindestens ein Modell mitsamt mindestens einem Modellparameter in Form einer Wahrscheinlichkeitsverteilung aufweisen, dass eine Zielvariable biochemischer Natur betrifft, insbesondere den Bräunungsgrad und/oder den Reifegrad, den Säuregehalt und/oder den Zuckergehalt oder die Konzentration von bestimmten Vitamine, oder oxidierter Fette. Es handeltsich hierbei um Zielvariablen zu gesundheitlich nachrangigen Eigenschaften.

Ebenfalls denkbare Zielvariablen von gesundheitlich nachrangiger Bedeutung, die jedoch für die Qualität des Lebensmittels dennoch erheblich sind, sind physikalischen Zielvariablen wie beispielsweise die Farbe, die Textur, der Wassergehalt, die Druckfestigkeit sowie die Trockenmasse, sowie eher subjektive oder aggregierte Zielvariablen wie der Geschmack oder die Frische.

Die Umgebungsparameter, die in der Zwillingsinstanz zugeordneter Weise abgespeichert werden, umfassen mindestens einen Umgebungsparameter, der den zeitlichen Verlauf eines Parameters der Umgebung angibt, der das Lebensmittel ausgesetzt ist. Hierbei kann es sich vor allem um eine Temperatur des Lebensmittels und/oder einer Umgebungstemperatur im Raum, in dem das Lebensmittel gelagert ist, handeln. Aber auch andere Umgebungsparameter wie beispielsweise die Umgebungsluftfeuchtigkeit im Raum, in dem das Lebensmittel gelagert ist, können für die Entwicklung der Zielvariablen relevant sein und daher erfasst und in der Zwillingsinstanz gespeichert werden. Ein weiterer in der Praxis mitunter erheblicher Umgebungsparameter, der ggf. in der Zwillingsinstanz zugeordneter Weise abgespeichert werden kann, betrifft die Zusammensetzung der das Lebensmittel während des Transportes und der Lagerung umgebenden Luft. Es kann sich beispielsweise um die Konzentration eines Spurengases ($CO_2$, Ethylen) handeln.

Die Erzeugung der digitalen Zwillingsinstanz erfolgt vorzugsweise zum frühestens Zeitpunkt, ab dem das Lebensmittel seinen verkaufsfertigen Zustand erreicht hat und ab dem eine Überwachung des mindestens einen Umgebungsparameters möglich ist.

Bei vielen Lebensmittelprodukten bedeutet dies, dass die Erzeugung der digitalen Zwillingsinstanz zum Zeitpunkt der Produktion erfolgt, beispielsweise bei Fleischprodukten im Zerlegebetrieb oder bei Fischereiprodukten während des Fangs oder zu mindest noch während der Fang auf dem Fischereiboot lagert.

Bei solchen Produkten, bei denen ab der Produktion der mindestens eine Umgebungsparameter, insbesondere die Lagertemperatur, erfasst werden kann, entsteht die digitale Zwillingsinstanz vorzugsweise als eine Art einfacher Kopie der zugehörigen Zwillingsvorlage, also ohne dass die Daten in der digitalen Zwillingsinstanz bereits in Hinblick auf das konkrete Lebensmittel oder seine Charge angepasst sind. Stattdessen sind es nur die für die Zwillingsvorlage charakteristischen Festlegungen und insbesondere die darauf basierenden Wahrscheinlichkeitsverteilungen der Modellparameter, die die Zwillingsinstanz gleichsam erbt. Die Zwillingsvorlage ist üblicherweise spezifisch für die Art des Lebensmittels und für den Produktionsbetrieb. Je nach Produkt kann es jedoch zusätzlich zweckmäßig sein, wenn die Zwillingsvorlage auch für einen vorgeschalteten Erzeugerbetrieb spezifisch ist, also beispielsweise Schweineschnitzel aus dem Zerlegebetrieb A betrifft, die von Schweinen aus dem Schweineaufzuchtbetrieb B stammen. Eine Messung von Zielvariablen bereits im Produktionsbetrieb zum Zwecke der Anpassung der zuvor erzeugten Zwillingsinstanz ist bei diesem Vorgehen üblicherweise nicht erforderlich. Dementsprechend sind die Wahrscheinlichkeitsverteilungen der Modellparameter der digitalen Zwillingsinstanz des Lebensmittels vorzugsweise bis mindestens zu dem Zeitpunkt unverändert, zu dem das Lebensmittel den Produktionsbetrieb verlässt.

Die Verfolgung von Umgebungsparameters bereits ab dem Zeitpunkt, zu dem das Produkt seinen verkaufsfertigen Zustand erreicht, ist jedoch in jedem Fall praktikabel. So ist insbesondere bei Importgütern, beispielsweise aus Südamerika nach Europa verschifftem Obst, beim Eintreffen in Europa eine große Unwahrscheinlichkeit in Hinblick auf die Zielvariablen gegeben. Die Erzeugung der digitalen Zwillingsinstanz alleine auf Basis einer für das Produkt und den Ursprung einheitlichen digitalen Zwillingsvorlage wäre in einem solchen Falle meist nicht sinnvoll. Stattdessen kann im Zuge des Imports, im Zuge einer Warenannahme und/oder eines Gefahrenübergangs einer Transportladung von Lebensmitteln eine Messung einer Zielvariablen durchgeführt. Die Erzeugung der digitalen Zwillingsinstanz erfolgt dann derart, dass mindestens eine Wahrscheinlichkeitsverteilung mindestens eines Modellparameters des mathematischen Modells der Zielvariablen in Abhängigkeit des Messergebnisses in der digitalen Zwillingsinstanz angepasst hinterlegt wird.

Vorzugsweise wird im Zuge der Handhabung des Lebensmittels bis zum Erreichen einer Verkaufsstätte und/oder an der Verkaufsstätte mindestens eine Messung einer Zielvariablen der Zwillingsinstanz des Lebensmittels vorgenommen. Dies kann in der oben genannten Art beispielsweise direkt beim Import geschehen. Alternativ oder zusätzlich kann es jedoch auch zu einem späteren Zeitpunkt in der Lieferkette geschehen, beispielsweise in Form einer Eingangsmessung beim Eingang bei der Verkaufsstätte.

Eine Messung einer Zielvariablen zur Anpassung der Zwillingsinstanz kann jedoch auch bei der Produktion eines Lebensmittels erfolgen, um die digitale Zwillingsinstanz noch besser an die Realität des repräsentierten Lebensmittels anzunähern.

Neben einer direkten Messung einer Zielvariablen der Zwillingsinstanz, können auch indirekte Daten zur Beschreibung der Fertigungsdetails sowie zur approximativen Bestimmung der Zielvariablen Verwendung finden. So könnte statt einer direkten Messung einer Keimkonzentration am Produkt am Ende der Fertigung, auch die Umgebungshygiene, Hygiene-Messungen an den Maschinen und in den Räumen der Fertigung mit herangezogen werden, wenn ausreichende Erfahrungswerte in Hinblick auf eine Korrelation zur Zielvariablen vorhanden sind. Weiterhin kann auch eine Methode aus dem Bereich der digitalen Bilderkennung Anwendung finden, um auf die Zielvariable rückschließen zu können.

In Abhängigkeit des Ergebnisses der Messung kann eine Aktualisierung der Zwillingsinstanz erfolgen, insbesondere eine Aktualisierung der Wahrscheinlichkeitsverteilung mindestens eines Modellparameters des mathematischen Modells der Zielvariablen. Insbesondere kann eine Anpassung eines Initialwertes der Zielvariablen auf Basis einer solchen Messung angepasst werden. Ein zu solchen Zweck anerkanntes Verfahren ist das Bayesian-Updating-Verfahren erfolgt, dessen Verwendung auch für den vorliegenden Fall vorgeschlagen wird.

Wenn auf Basis einer erfolgten Messung die Wahrscheinlichkeitsverteilung eines Modellparameters in der Zwillingsinstanz angepasst wird, ist es von Vorteil, wenn die entsprechende zuvor geltende Wahrscheinlichkeitsverteilung des Modellparameters zum Zwecke der späteren Nachvollziehbarkeit im Speicher belassen wird, so dass der Zwillingsinstanz entnommen werden kann, zu welchem Zeitpunkt Wahrscheinlichkeitsverteilungen in Hinblick auf die Zielvariable unter Berücksichtigung welcher Wahrscheinlichkeitsverteilung der Modellparameter stattgefunden hat.

Neben der Prüfung und gegebenenfalls vorgesehenen Aktualisierung der Zwillingsinstanz auf Basis einer Messung kann auch vorgesehen sein, dass auf Basis der Ergebnisse dieser Messung sowie einer Mehrzahl weiterer Messungen von Lebensmitteln, deren Zwillingsinstanzen von der gleichen Zwillingsvorlage abgeleitet wurden, eine Aktualisierung der Zwillingsvorlage erfolgt.

Eine solche Aktualisierung kann vollständig automatisch erfolgen. Dies ist jedoch in der Regel nicht bevorzugt. Stattdessen wird es als zweckmäßig angesehen, wenn die durch derartige Messungen ermittelten Daten von einem Fachmann gesichtet werden und dieser unter Einbeziehung seines Fachwissens beurteilt, inwieweit die Messdaten eine Anpassung des in der Zwillingsvorlage hinterlegten mathematischen Modells und/oder der Wahrscheinlichkeitsverteilung mindestens eines Modellparameters zweckmäßig erscheinen lassen.

Neben der Aktualisierung der Zwillingsinstanz oder gegebenenfalls sogar der Zwillingsvorlage kann die Messung, insbesondere sofern sie einen gesundheitlich bedenklichen Wert der Zielvariable ergibt, der bezogen auf die auf Basis der Zwillingsinstanz ermittelte Wahrscheinlichkeit der Zielvariable unwahrscheinlich ist, herangezogen werden, um Auswirkungen auch auf die Zwillingsinstanzen anderer Lebensmittel zu bewirken, insbesondere anderer Lebensmittel, die der gleichen Charge wie der des gemessenen Lebensmittels entstammen.

So kann insbesondere eine Anpassung anderer Lebensmittel in Hinblick auf die Wahrscheinlichkeitsverteilung der Modellparameter erfolgen. Sofern sich aus der gemessenen Zielvariablen eine relevante gesundheitliche Gefahr ergibt, kann gegebenenfalls sogar das Erzeugung einer Warnmitteilung zweckmäßig sein, insbesondere einer Warnmitteilung, die Zwillingsinstanzen anderer Lebensmittel zugeordnet wird, so dass bei der Abfrage von Zielvariablen dieser Zwillingsinstanzen anderer Lebensmittel die entsprechende Warnmitteilung ausgegeben wird.

Gegebenenfalls kann die Messung auch verursachen, dass eine Haltbarkeitsinformation unter Berücksichtigung der vorgesehenen oder alternativen Verwendungsarten erzeugt wird, der zu entnehmen ist, wie lange das Lebensmittel oder andere Lebensmittel der gleichen Charge trotz bedenklicher Messwerte für die jeweilige Verwendungsart noch geeignet ist.

Die Verwendung der digitalen Zwillingsinstanz zur Abschätzung der Werte der jeweiligen Zielvariablen bei einem konkreten Lebensmittel oder dessen Charge findet insbesondere vor dem Verkauf durch an der Lieferkette beteiligte Personen statt. So kann beispielsweise Personal an der Verkaufsstätte die digitalen Zwillinge eingehender Lebensmittel prüfen, um dies ggf. zurückweisen zu können oder mit angepassten Endkundeninformationen zur Haltbarkeit versehen zu können.

Auch erlauben es die Zwillingsinstanzen unterschiedlicher Lebensmittel oder unterschiedlicher Chargen der Lebensmittel, dass Personal entlang der Lieferkette Entscheidungen zur Priorität bei der Handhabung der Lebensmittel treffen. So kann beispielsweise eine Transportreihenfolge bei begrenzten Transportkapazitäten von den jeweiligen digitalen Zwillingen und den sich daraus ergebenden Wahrscheinlichkeitsverteilungen in Hinblick auf die jeweiligen Zielvariablen bestimmt werden. Auch die Preisgestaltung an der Verkaufsstätte kann von der Wahrscheinlichkeitsverteilung der Zielvariablen abhängig gemacht werden.

Des Weiteren kann die Güte einer Transportkette im Hinblick auf Kühlung oder Luftfeuchtigkeit quantifiziert werden. Dazu kann man die ermittelte Haltbarkeit der Lebensmittel aufgrund der tatsächliche gemessenen Umgebungsvariablen ins Verhältnis setzen zu der Haltbarkeit, die man auf Grundlage der vertraglich vereinbarten Transportbedingungen zu erwarten hätte. Auch ist es möglich, vertragliche Vereinbarungen mit Transportunternehmen zu treffen, die eine mindestens zu erzielende Wahrscheinlichkeitsverteilung einer oder mehrere Zielvariablen vorgeben. Hierdurch wird es in der Praxis einfacher, schwer vermeidbare Abweichungen von durchgehend idealen Lagerbedingungen richtig in Hinblick auf ihre Auswirkungen auf die Haltbarkeit einzuschätzen und zu beurteilen, ob die Handhabung durch das Transportunternehmen dennoch noch vertragsgemäß sind und die gewünschte Qualität des Lebensmittels gegeben ist.

Ein weiterer Nutzen kann jedoch auch darin bestehen, dass die Ermittlung der Wahrscheinlichkeit in Hinblick auf die mindestens eine Zielvariable unter Verwendung der Zwillingsinstanz auch nach Auslösung über ein Abfragegerät eines Kunden oder Verbrauchers an der Verkaufsstätte oder nach Erwerb des Lebensmittels erfolgen kann. Dies gestattet es dem Kunden, seine Kaufentscheidung von der Wahrscheinlichkeitsverteilung der Zielvariablen abhängig zu machen oder beim Verbrauch der Lebensmittel in seinem Kühlschrank zu berücksichtigen, ab wann welche Lebensmittel mit erhöhter Wahrscheinlichkeit nicht mehr ohne gesundheitliche Bedenken verzehrt werden dürfen.

Das Abfragegerät kann insbesondere ein Mobilfunkgerät sein, welches über die Kamera oder anderweitige Sensoren das Scannen eines Lebensmittels an der Verkaufsstätte ermöglicht.

Auf dem Abfragegerät des Kunden wird vorzugsweise angezeigt, ob eine oder mehrere Zielvariablen sich mit vorgegebener Wahrscheinlichkeit in einem gesundheitlich unbedenklichen Bereich befinden. Alternativ oder zusätzlich kann auf dem Abfragegerät des Kunden angezeigt werden, mit welcher Wahrscheinlichkeit sich eine oder mehrere Zielvariablen in einem gesundheitlich unbedenklichen Bereich befinden.

Vorzugsweise ist weiterhin vorgesehen, dass die Ermittlung der Wahrscheinlichkeit in Hinblick auf die mindestens eine Zielvariable unter Einbeziehung von prognostizierten oder gemessenen Daten zum mindestens einen Umgebungsparameter der Zwillingsinstanz erfolgt, wobei hierfür durch den Kunden oder Verbraucher insbesondere Daten zur Verfügung gestellt werden, die wiedergeben, unter welchen Bedingungen das Lebensmittel gelagert wurde oder werden wird, und/oder Daten zur Verfügung gestellt werden, die wiedergeben, wie lange und/oder unter welchen Bedingungen das Lebensmittel transportiert wurde oder werden wird, bis es eine Kühleinrichtung des Kunden oder Verbrauchers erreicht. Entsprechende Daten können vom Kunden voreingestellt oder anderweitig zur Verfügung gestellt im Abfragegerät auch bereits hinterlegt sein.

Im Falle, dass eine Warnmitteilung der Zwillingsinstanz zugeordnet ist, wird diese vorzugsweise auf dem Abfragegerät ausgegeben. Der Kunde kann somit sogar nach Kauf des Lebensmittels informiert werden, wenn andere Lebensmittel der gleichen Charge bei Messungen eine erhöhte gesundheitliche Gefährdung nahegelegt haben, die die Annahme rechtfertigt, dass auch andere Lebensmittel der Charge betroffen sein können.

Die Erfindung betrifft darüber hinaus ein Computerprogrammprodukt oder ein Computersystem mit einem Computerprogrammprodukt, wobei das Computerprogrammprodukt Befehle umfasst, die bei der Ausführung des Programms durch einen Computer diesen veranlasst, das Verfahren nach einem der vorstehenden Ansprüche durchzuführen. Ein Computersystem hierfür umfasst verschiedene Teilkomponenten an verschiedenen Orten einer Lieferkette, wie es exemplarisch anhand der folgenden Ausführungsbeispiele verdeutlicht ist.

KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.

Fig. 1 zeigt den Weg eines Lebensmittels von der Produktionsstätte bis in den Kühlschrank eines Kunden sowie den derweil stattfindenden Informationsaustausch mit einem Server zur Erzeugung und Manipulation einer digitalen Zwillingsinstanz.

Fig. 2A bis 2F zeigen die digitale Zwillingsinstanz ausgehend von der Erzeugung im Zuge der Produktion des Lebensmittels.

Fig. 3A bis 3C zeigen die Wahrscheinlichkeitsverteilungen von Modellparametern der digitalen Zwillingsinstanz.

Fig. 3D zeigt zwei exemplarische Temperaturverläufe, denen das durch die Zwillingsinstanz repräsentierte Lebensmittel unterliegen könnte.

Fig. 3E zeigt, wie sich nach 240 Stunden unterschiedliche Wahrscheinlichkeitsverteilungen in Hinblick auf die Keimbelastungam Lebensmittel in Abhängigkeit der unterschiedlichen Temperaturverläufe gemäß Fig. 3D ergeben.

Fig3F zeigt, wie sich aus den Wahrscheinlichkeitsverteilungen Aussagen zur vermuteten Keimbelastung mit unterschiedlichen Wahrscheinlichkeiten ableiten lassen.

DETAILLIERTE BESCHREIBUNG DERAUSFÜHRUNGSBEISPIELE

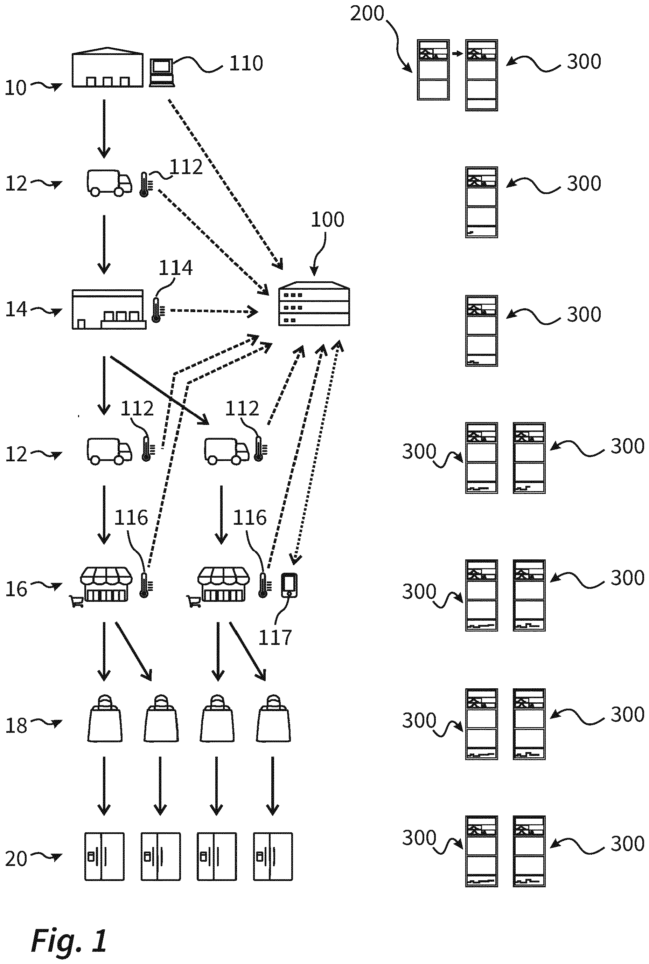

Fig. 1 zeigt den grundsätzlichen Weg eines Lebensmittels. Schweineschnitzel werden in einer Produktionsstätte 10, beispielsweise einem Zerlegebetrieb, hergestellt, von hier aus mittels beispielsweise Lastkraftwagen 12 und ggf. unter Zwischenlagerung in einem Kühlhaus 14 zu Verkaufsstätten 16 gebracht. Hier wird das Produkt von Kunden erworben, von diesen in Taschen 18 in ihre jeweiligen Wohnstätten gebracht und dort zunächst bis zur Zubereitung oder bis zum Verzehr in Kühlschränken 20 gelagert.

Um zu jedem Zeitpunkt in der Lage zu sein, Eigenschaften des Lebensmittels zu beurteilen, wird das Lebensmittel auf dem in Fig. 1 skizzierten Weg durch zunächst eine digitale Repräsentation 300 begleitet, die gleichsam einen digitalen Zwilling des Lebensmittels darstellt. Dieser digitale Zwilling, im weiteren als digitale Zwillingsinstanz 300 bezeichnet, spiegelt wieder, wie mindestens eine Zielvariable, vorzugsweise eine Mehrzahl von Zielvariablen, sich über die Lebenszeit des von ihm repräsentierten Lebensmittels verändern.

Unter einer Zielvariablen wird dabei eine Größe verstanden, die für die Qualität und insbesondere die Verzehreignung des Lebensmittels maßgeblich ist. Hierbei kann es sich insbesondere um gesundheitlich relevante Größen wie beispielsweise eine Belastung mit Bakterien handeln. Es kann sich jedoch auch um eine gesundheitlich nachrangige Größe wie beispielsweise die Konsistenz oder das Aroma handeln. Die Einheiten von Zielvariablen können eindeutig definierte Einheiten sein wie beispielsweise die Anzahl der koloniebildenden Einheiten einer bestimmten Keimsorte pro Gewichtseinheit des Lebensmittels. Es können jedoch auch willkürlich gewählte Punkteeinheiten sein, beispielsweise ein Wert in einem Intervall zwischen 0 und 100, der die Geschmacksqualität wiederspiegelt, wobei 100 Punkte die höchste Geschmacksqualität repräsentiert.

Die genannte digitale Repräsentation, die digitale Zwillingsinstanz, wird insbesondere auf einem Zentralserver 100 verwaltet, auf den die verschiedenen im Weiteren noch beschriebenen Systeme Zugriff haben, insbesondere über das Internet, um die digitalen Zwillingsinstanz 300 zu erzeugen bzw. um Daten dieser digitalen Zwillingsinstanz 300 abzufragen oder zu ergänzen. Bei dem Zentralserver 100 handelt es sich in der heute üblichen Art vorzugsweise nicht um einen spezifischen Rechner, sondern üblicherweise um eine Serverinstanz oder einen virtuellen Server, die in einem Rechenzentrum auf einer Vielzahl von zusammenwirkenden Rechnern arbeitet. Eine derartige Infrastruktur wird auch häufig als Cloud bezeichnet. Sie gestattet es, das System einfach skalierbar zu gestalten. Zur sprachlichen Vereinfachung wird im Weiteren dennoch von einem Zentralserver gesprochen, wenn diese zentrale Datenverwaltung gemeint ist.

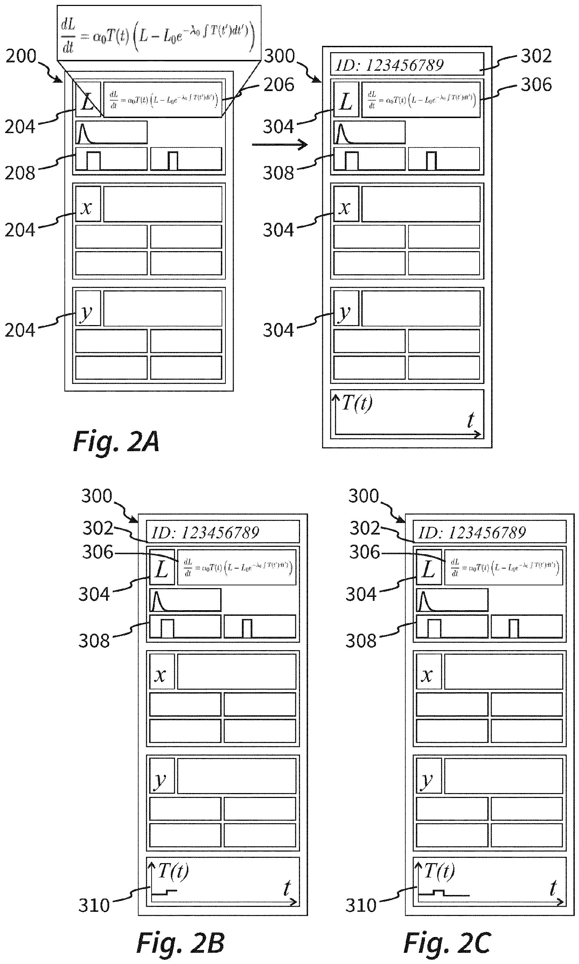

Es ist vorgesehen, dass eine Vorlage 200 für die genannten digitalen Zwillingsinstanzen existiert, die unabhängig von einem konkreten Lebensmittel bereits im Vorfeld erzeugt wurde. Eine solche digitale Zwillingsvorlage 200 bezieht sich vorzugsweise auf einen bestimmten Lebensmitteltyp und eine bestimmte Produktionsstätte. Die digitale Zwillingsvorlage 200, die in Fig. 2A auf der linken Seite dargestellt ist, kann im Vorfeld der Produktion eines Lebensmittels auf Basis von Expertenwissen und deren Erfahrung sowie auf Basis von historischen Messdaten der Zielvariablen modelliert werden und ggf. überarbeitet werden. Sie kann jedoch auch automatisch aus Daten der Vergangenheit abgeleitet und/oder anhand von neueren Daten automatisch aktualisiert werden.

Die digitale Zwillingsvorlage 200 umfasst primär zweierlei: Zum einen mindestens ein mathematisches Modell 206 zur Berechnung mindestens einer Zielvariable 204, wobei dieses mathematische Modell 206 mindestens einen Modellparameter und mindestens einen Umgebungsparameter umfasst, zum anderen eine Wahrscheinlichkeitsverteilung 208 für den mindestens einen Modellparameter.

Üblicherweise ist eine digitale Zwillingsvorlage 200 für die Abschätzung von mehreren Zielvariablen 204 vorgesehen. In diesem Falle weist sie je Zielvariable 204 ein eigenes mathematisches Modell 206 und je mathematischem Modell mindestens einen Modellparameter mit einer Wahrscheinlichkeitsverteilung 208 auf.

Vorliegend wird jedoch zum Zwecke der vereinfachten Verdeutlichung eine digitale Zwillingsvorlage 200 betrachtet, zu der in Hinblick auf die Verfolgung einer Zielvariablen 204 beschrieben ist. Die hier betrachtete digitale Zwillingsvorlage 200 ist spezifisch für ein bestimmtes Lebensmittel, vorliegend exemplarisch Schweineschnitzel, und eine bestimmte Produktionsstätte, vorliegend einen fiktiven Zerlegebetrieb A, gekennzeichnet in Fig. 1 durch die Bezugsnummer 10. Grundsätzlich könnten die Zwillingsvorlagen 200 jedoch noch differenzierter vorgehalten werden. So können beispielsweise für unterschiedliche Mastbetriebe unterschiedliche Zwillingsvorlagen 200 vorgesehen sein.

Die hier betrachtete Zielvariable 204, zu deren Ermittlung die digitale Zwillingsvorlage 200 unter anderem vorgesehen ist, ist die Listerienkonzentration L. Listerien sind Bakterien, die in der Natur allgegenwärtig vorkommen und sich von totem organischen Material ernähren. Somit sind Listerien auch nach Schlachtung von Tieren auf den hieraus gewonnenen Lebensmitteln, vorliegend auf Schweineschnitzeln, zu finden. Als Einheit für solche Bakterien wird die Anzahl der koloniebildenden Einheiten pro Gramm des Lebensmittels verwendet (CFU/g).

Das in der hier betrachteten Zwillingsvorlage 200 verwendete mathematische Modell lautet wie folgt:

$$\frac{dL}{dt} = \alpha_0 T(t) \left( L - L_0 e^{-\lambda_0 \int_{t_0}^{t} T(t')dt'} \right)$$

$$\frac{dL}{dt} = \alpha_0 T(t) \left( L - L_0 e^{-\lambda_0 \int T(t')dt'} \right)$$

Es handelt sich um eine Differentialgleichung, die auf der linken Seite der Gleichung die erste Ableitung der Listerienkonzentration nach der Zeit aufweist.

Wie sich dem Modell entnehmen lässt, weist dieses weiterhin drei Modellparameter auf, nämlich die Modellparameter $\alpha_0$, $L_0$ und $\lambda_0$. Mindestens einer dieser Modellparameter, vorzugsweise alle drei Modellparameter, liegen nicht in Form konkreter Werte vor, sondern in Form einer Wahrscheinlichkeitsverteilung. Die drei Modellparameter stehen dabei für folgende Informationen:

$L_0$ ist die Wahrscheinlichkeitsverteilung der initialen Keimbelastung mit Listerien, die Schweineschnitzel der Produktionsstätte 10 aufweisen, vorliegend also des Zerlegebetriebs.

$\lambda_0$ ist die Wahrscheinlichkeitsverteilung der Übergangsrate pro Temperatureinheit, mit der die Listerien auf dem Schnitzel im Mittel aus einer inaktiven Lag-Phase in die Wachstums-Phase übergehen.

$\alpha_0$ ist die Wahrscheinlichkeitsverteilung der Wachstumsrate pro Temperatureinheit, mit der sich die Listerien auf dem Schweineschnitzel vermehren, sofern sie sich in der Wachstumsphase befinden.

In der Praxis finden voraussichtlich komplexere mathematische Modelle mit mehr Modellparametern Anwendung. Für die Zwecke dieser Beschreibung reicht jedoch das genannte vereinfachte mathematische Modell.

Mit der Produktion einer Charge von Schweineschnitzeln in der Produktionsstätte 10 wird ausgehend von der Zwillingsvorlage 200 eine digitale Zwillingsinstanz 300 für die gesamte Charge erzeugt, wie in Fig 2A verdeutlicht wird. Dies bedeutet, dass von einem Rechner 110 in der Produktionsstätte aus, bei dem es sich beispielsweise auch im einen in einen Barcode-Scanner integrierten Rechner handeln kann, eine Aufforderung an den Zentralserver 100 übermittelt wird, einen neuen Zwillingsinstanz-Datensatz im Speicher zu erzeugen. Der Zentralserver 100 erzeugt daraufhin die Zwillingsinstanz 300 auf Basis der Zwillingsvorlage 200, wobei das mathematische Modell bzw. die mathematischen Modelle sowie die zugehörigen Wahrscheinlichkeitsverteilungen der zugehörigen Modellparameter von der Zwillingsvorlage 200 übernommen werden. Dabei ist ein Kopieren des mathematischen Modells 206 bzw. der mathematischen Modelle 206 und der Wahrscheinlichkeitsverteilungen der Modellparameter in die Zwillingsinstanz 300 möglich. Bevorzugt ist es allerdings, dass lediglich eine Referenzierung stattfindet, also die Zwillingsinstanz 300 einen Verweis auf die Zwillingsvorlage 200 enthält.

Neben dem mathematischen Modell 306 bzw. den mathematischen Modellen 306 mitsamt Wahrscheinlichkeitsverteilung 308 der Modellparameter weist die Zwillingsinstanz 300 noch eine eindeutige Kennung 302 auf, um eine eindeutige Verbindung zum Lebensmittel, vorliegend zunächst primär der Charge, herzustellen. Bei der eindeutigen Kennung 302 kann es sich um eine Chargennummer handeln. Denkbar sind jedoch auch andere Kennungen. So könnten beispielsweise eine oder mehrere Paletten-Identifikationsnummern als Kennung herangezogen werden.

Neben der Kennung können weitere Informationen in der Zwillingsinstanz abgelegt werden, welche für die Charge wesentliche Informationen darstellen und insbesondere vom Rechner 110 zusammen mit der Aufforderung zur Erzeugung der digitalen Zwillingsinstanz an den Zentralserver 100 übermittelt werden, so beispielsweise der Betrieb, aus dem das Schweinefleisch ursprünglich stammt und/oder das Datum der Herstellung im Zerlegebetrieb und/oder das Datum der Schlachtung.

Weiterhin ist die Zwillingsinstanz 300 mit Speicherplatz versehen, der es gestattet, Umgebungsparameter und deren zeitliche Entwicklung hierin abzulegen. Vorliegend ist der einzige Umgebungsparameter 310, der für das mathematische Modell zur Ermittlung der Listerienkonzentration erforderlich ist, der Parameter der Temperatur T und ihrer zeitlichen Änderung.

Beginnend mit der Produktion in der Produktionsstätte 10 werden die jeweils gegebenen Temperaturdaten als maßgeblicher Umgebungsparameter an den Zentralserver 100 übertragen und in der Zwillingsinstanz 300 abgelegt. Die Temperaturdaten können dabei beispielsweise automatisiert in Kühlräumen der Produktionsstätte 10, des Kühlhauses 14 und der Verkaufsstätte 16 erfasst werden und anhand der zuvor registrierten Chargennummer der darin gelagerten Lebensmittel an den Server 100 übertragen werden. Gleiches gilt auch für den Transport bis zur Verkaufsstätte in den Lastkraftwagen 12. Daneben ist natürlich grundsätzlich auch möglich, entlang des Pfades partiell lediglich Annahmen zu treffen, insbesondere basierend auf Erfahrungswerten oder beispielsweise an Kühlaggregaten in der Kühlsteuerung hinterlegten Werten, ohne dass es sich um tatsächlich gemessene Temperaturwerte handelt. Die erfassten oder bekannten Temperaturdaten werden unter Angabe der Chargennummer oder eines anderen eindeutigen Kennzeichens von Dateneinrichtungen 112,114,116 ebenfalls an den Server 100 übermittelt und als ergänzende Daten zur Zwillingsinstanz genommen.

Fig. 2B und 2C verdeutlichen, wie die Temperaturdaten der Zwillingsinstanz durch entsprechende Daten des Transports im Lastkraftwagen 12 und des Kühlhauses 14 ergänzt werden.

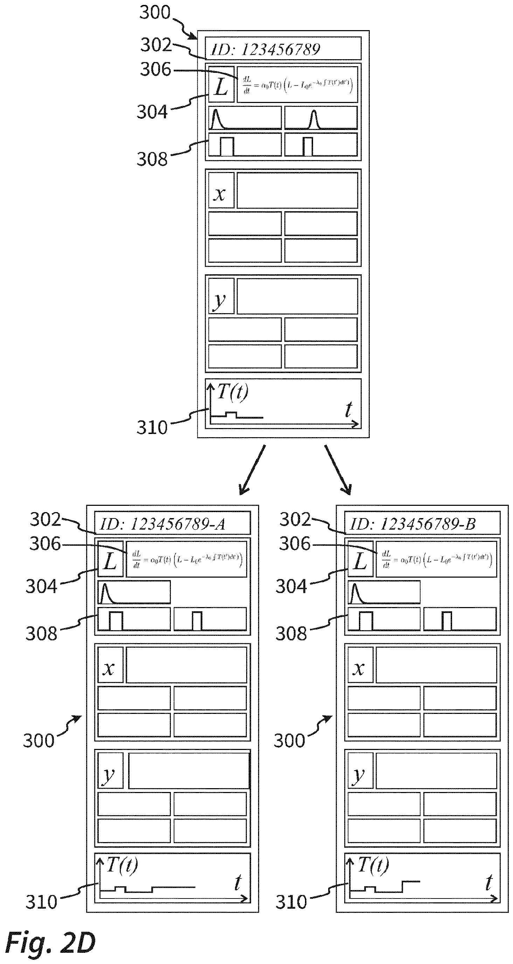

Die Schweineschnitzel der Charge werden vom Kühlhaus 14 aus durch Lastkraftwagen 12 in unterschiedliche Verkaufsstätten transportiert, wobei in Fig. 1 exemplarisch zwei solche Verkaufsstätten dargestellt sind. Dadurch, dass sich die Wege unterschiedlicher Schweineschnitzel der Charge hierdurch trennen, reicht auch eine einzige Zwillingsinstanz nicht mehr aus. Die Zwillingsinstanz wird daher entsprechend der Anzahl der Wege vervielfacht, wobei dies in Fig. 1 und Fig. 2D durch zwei digitale Zwillingsinstanzen 300 ab dem Transport zu den Verkaufsstätten 16 verdeutlicht wird. Die zur Identifizierung der Zwillingsinstanz 300 bis hierhin genutzte Chargennummer wird um ein eindeutiges Kennzeichen in Hinblick auf den weiteren Weg ergänzt, beispielsweise um eine Kennung der Verkaufsstätte 16. Wie oben erläutert, könnte aber auch schon die noch einheitliche Zwillingsinstanz 300 mehrere Kennungen beinhalten, insbesondere alle Kennungen der Paletten, auf die die Charge transportiert wird. In diesem Falle können nach dem Duplizieren der Zwillingsinstanz die beiden Zwillingsinstanzen 300 als Kennung jeweils jene Palettenkennungen beinhalten, die zu Paletten gehören, die an jeweils eine Verkaufsstätte weitergeliefert werden.

Die zwei Zwillingsinstanzen 300 werden ab der Trennung jeweils auf dem Server 100 mit Temperaturdaten ergänzt, wobei diese Temperaturdaten sich aufgrund der unterschiedlichen Wege voneinander unterscheiden.

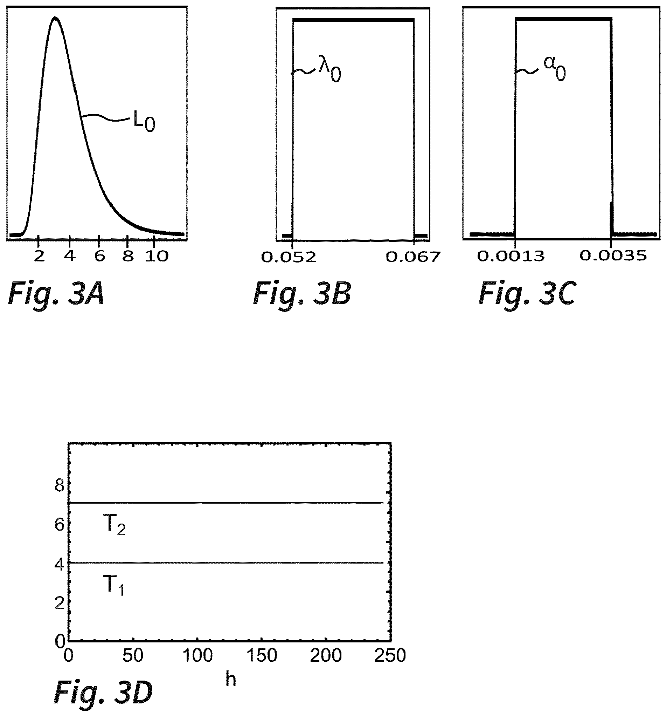

Ausgehend von den in den verschiedenen Phasen des Weges des Lebensmittels beginnend mit er Produktionsstätte jeweils hinzukommenden Temperaturdaten ist jederzeit eine Auswertung des Lebensmittels auf Basis der Zwillingsinstanz 300 möglich. Hierbei werden die Zielvariablen der Zwillingsinstanz 300 errechnet, wobei das Ergebnis dieser Berechnung jeweils eine Wahrscheinlichkeitsverteilung ist. Dies wird im Weiteren näher anhand der Fig. 3A bis 3E verdeutlicht:

Fig. 3A bis 3C zeigt die Verteilungen der Modellparameter $L_0$, $\lambda_0$ und $\alpha_0$, die aus der Zwillingsvorlage 200 stammend für die Erstellung des Zwillingsmodells herangezogen wurden. Wie bereits erwähnt, sind diese Wahrscheinlichkeitsverteilungen in der Zwillingsinstanz 300 vorzugsweise und insbesondere vorzugsweise anfänglich lediglich referenziert und verweisen auf die Zwillingsvorlage 200. Es ist zu ersehen, dass die Wahrscheinlichkeitsverteilungen der Übergangsrate $\lambda_0$ und der Wachstumsrate $\alpha_0$ rechteckförmig sind, dass also jeweils mit gleicher Wahrscheinlichkeit ein Übergangswert zwischen 0,052 und 0,067 in Hinblick auf die Übergangsrate $\lambda_0$ und von 0,0013 bis 0,0035 in Hinblick auf die Wachstumsrate $\alpha_0$ gegeben ist. Die genannte Rechteckform der Wahrscheinlichkeitsparameter stellt vermutlich nicht die reale Wahrscheinlichkeitsverteilung dar, sondern ist der Tatsache geschuldet, dass lediglich ein Konfidenzintervall bekannt ist, innerhalb dessen die Wahrscheinlichkeit größer Null ist, wobei die exakte Verteilung jedoch nicht bekannt ist und für eine Abschätzung der Listerien mittels des mathematischen Modells 306 auch nicht erforderlich ist. Die initiale Keimbelastung $L_0$ ist dagegen insbesondere auf Basis früherer Messungen genauer bekannt und weist dagegen eine uneinheitlichere Wahrscheinlichkeitsverteilung mit einem Peak bei etwa 3 CFU/g auf, die in etwa einer logarithmischen Normalverteilt gleicht. Fig. 3D zeigt einen ersten möglichen Temperaturverlauf $T_1$ und einen zweiten möglichen Temperaturverlauf $T_2$ innerhalb der ersten 240 Stunden nach Herstellung des Schweineschnitzels. Die Temperaturverläufe sind dabei exemplarisch stark vereinfacht, denn betrachtet werden konstante Temperaturen von $T_1$=4°C und $T_2$=7°C. In der Praxis würden hier komplexere Temperaturverläufe einbezogen werden.

Unter Verwendung des Temperaturverlaufs $T_1$ bzw $T_2$ sowie der Modellparameter $L_0$, $\lambda_0$ und $\alpha_0$ der Zwillingsinstanz 300 kann jederzeit die Formel des mathematischen Modells 306 gelöst werden. Da die Modellparameter oder erfindungsgemäß zumindest ein Modellparameter in Form einer Wahrscheinlichkeitsverteilung 308 vorliegt, kann die Formel des mathematischen Modells 306 nicht durch einmalige Anwendung gelöst werden. Stattdessen können verschiedene Werte für die jeweiligen Modellparameter in separaten Rechenschritten herangezogen und im Ergebnis unter Berücksichtigung ihrer jeweiligen Wahrscheinlichkeit einbezogen werden. In der Praxis ist dieser Weg jedoch nicht geschickt, da die gleichen Ergebnisse auch mit geringerem Rechenbedarf mit geeigneten statistischen Methoden, insbesondere der Monte-Carlo-Methode, erreicht werden können.

Auch das Monte-Carlo Verfahren kann fallweise nicht effizient genug sein, beispielsweise wenn viele Nutzeranfragen in nahezu Echtzeit bearbeitet werden müssen und dies mit einer zu großen Latenzzeit zwischen Anfrage und Ergebnis einherginge. Dies ist insbesondere im Hinblick auf den Endkunden und der mobilen App bedeutsam. Um eine entsprechend kurze Antwortzeit zu gewährleisten, kann alternativ auf andere mathematische Verfahren wie spektrale Entwicklungen (Generalized Polynomial Chaos Expansion) oderstochastische Kollokation zurückgegriffen werden.

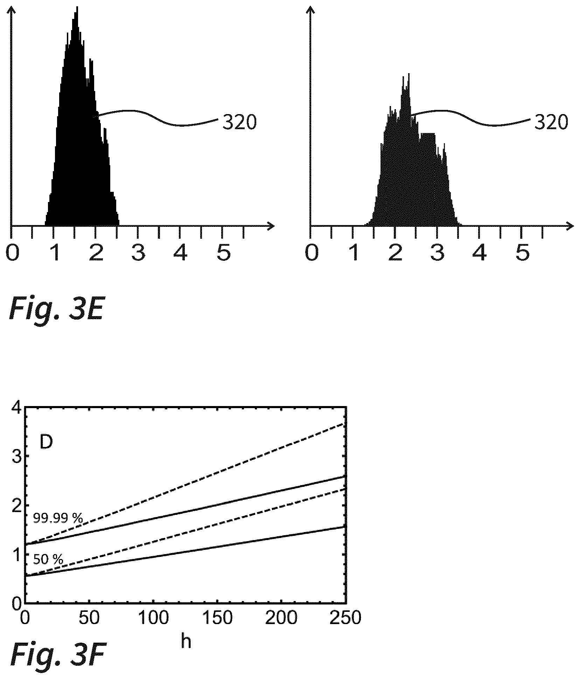

Ergebnis ist wiederum eine Wahrscheinlichkeitsverteilung, nämlich eine der Wahrscheinlichkeitsverteilungen, die in Fig. 3E dargestellt sind. Die linke Wahrscheinlichkeitsverteilung ist dabei die Wahrscheinlichkeitsverteilung zur Listerienkonzentration L, die sich bei einer durchgehenden Temperatur von $T_1$=4°C über einen Zeitraum von 240 Stunden einstellt. Die rechte Wahrscheinlichkeitsverteilung ist jene, die sich bei einer durchgehenden Temperatur von $T_2$=7°C über einen Zeitraum von 240 Stunden einstellt.

Diese Wahrscheinlichkeitsverteilungen der Fig. 3E sind jedoch üblicherweise nicht der letzte Schritt der Auswertung. Vielmehr kann eine solche Wahrscheinlichkeitsverteilung genutzt werden, um zu ermitteln, mit welcher Wahrscheinlichkeit das Schweineschnitzel eine Belastung mit der hier betrachteten Zielvariable der Listerien unterhalb eines vorgegebenen Wertes aufweist, so dass für unterschiedliche Fragestellungen Feststellungen getroffen werden können. So zeigt beispielsweise die Figur 3f die Listerienkonzentration auf der Y-Achse und verdeutlicht mit den Graphen, mit welcher Wahrscheinlichkeit über der Zeit jeweils 50% bzw. jeweils 99,99% unterhalb der jeweiligen Listerienbelastung liegen. Die gestrichelten Verläufe stehen dabei für eine Lagerung bei konstant 7°C, während die durchgezogenen Verläufe für eine Lagerung bei konstant 4°C stehen.

Für das beschriebene Verfahren sind direkte Messungen der mindestens einen Zielvariable, vorliegend also insbesondere der Listerienkonzentration, an derfraglichen Charge grundsätzlich nichterforderlich. Alleine auf Basis des digitalen Zwillings und des darin enthaltenen mathematischen Modells zur Listerienkonzentration sowie der hinterlegten Modellparameter lässt sich bei bekannter Historie der Umgebungsparameter, vorliegend der Temperatur, eine Abschätzung zur gegenwärtigen Listerienkonzentration in der beschriebenen Art durchführen.

Dennoch sind Messungen der Zielvariablen, beim vorliegenden Beispiel der Listerienkonzentration, zweckmäßig und finden üblicherweise entlang der Vertriebskette statt, insbesondere bei Auslieferung aus der Produktionsstätte 10 oder dem Kühlhaus 14 oder bei Anlieferung beim Kühlhaus 14 oder der Verkaufsstätte 16. Je nach Art der Messung kann diese dann zur Aktualisierung der betroffenen Zwillingsinstanz und/oder zur mittelbaren Aktualisierung der Zwillingsvorlage herangezogen werden.

Einmalige Messungen an nur einem oder wenigen Schweineschnitzeln der Charge sollten für den Fall, dass die gemessene Zielvariable, vorliegende die Listerienkonzentration, in dem Bereich liegt, den der digitale Zwilling 300 nahelegt, keinen oder keinen nennenswerten Einfluss auf die Zwillingsinstanz oder die Zwillingsvorlage nehmen.

Wird jedoch eine umfangreichere Messreihe durchgeführt, die geeignet ist, statistische Abweichungen gegenüber dem realen Wert der Listerienkonzentration L weitgehend auszuschließen und ist die gemessene Listerienkonzentration L nicht in dem auf Basis der Zwillingsinstanz plausiblen Bereich, wie er sich exemplarisch aus Fig. 3E ergibt, so kann für diesen Fall eine Anpassung der Zwillingsinstanz 300 vorgesehen sein. Die Zwillingsinstanz wird dabei in geeigneter Art aktualisiert, dass zukünftige Berechnungen der Listerienkonzentration da Ergebnis der Messung miteinbeziehen. Die Aktualisierung wird dabei vorzugsweise eine Korrektur der Wahrscheinlichkeitsverteilung der initialen Keimbelastung $L_0$ mit Listerien sein. Da es sich bei der aktualisierten Wahrscheinlichkeitsverteilung der initialen Keimbelastung $L_0$ um einen Modellparameter handelt, der nach der Anpassung nun nicht mehr mit der entsprechenden Wahrscheinlichkeitsverteilung der zugrundeliegenden Zwillingsvorlage übereinstimmt, wird die Zwillingsinstanz die geänderte Wahrscheinlichkeitsverteilung der initialen Keimbelastung $L_0$ vorzugsweise nicht mehr referenzieren, sondern unmittelbar als zwillingsinstanzspezifische Daten enthalten.

Für die Aktualisierung der zuvor auf der Zwillingsvorlage basierenden Wahrscheinlichkeitsverteilung der initialen Keimbelastung $L_0$ dahingehend, dass diese Wahrscheinlichkeitsverteilung anschließend auch die Werte der Messung miteinbezieht, stehen geeignete statistische Methoden zur Verfügung, so insbesondere das Bayesian-Updating-Verfahren zur Verfügung.

Die Aktualisierung der Wahrscheinlichkeitsverteilung und damit ein Ersetzen der ursprünglichen Wahrscheinlichkeitsverteilung müssen nicht unmittelbar stattfinden. Denkbar ist auch, dass zunächst nur die Messergebnisse in der Zwillingsinstanz 300 abgelegt werden und die Anpassung der Wahrscheinlichkeitsverteilung erst zu einem späteren Zeitpunkt erfolgt, insbesondere wenn die Wahrscheinlichkeitsverteilung der Listerienkonzentration auf Basis der Zwillingsinstanz 300 abgefragt wird.

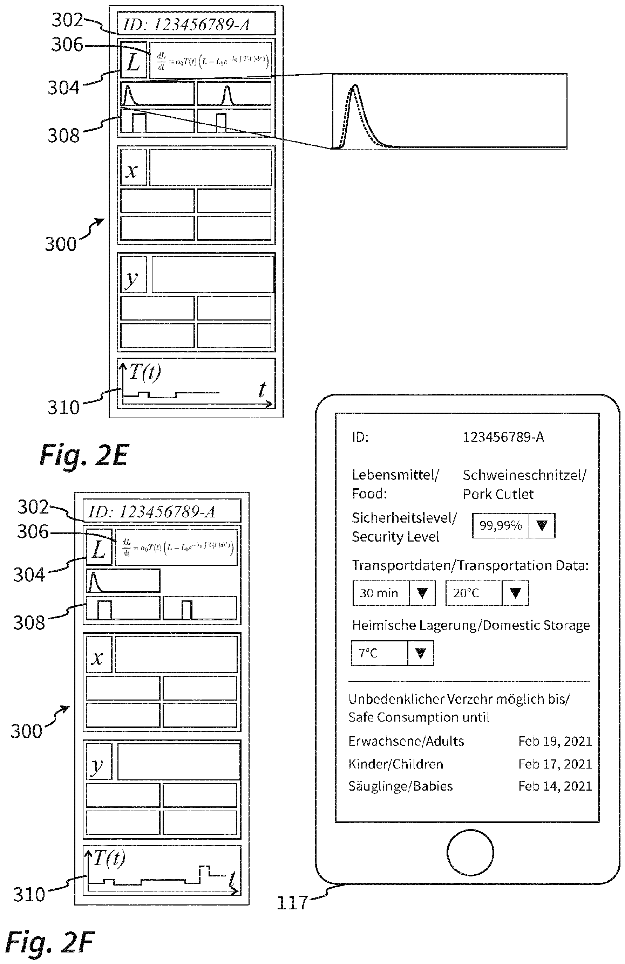

Fig. 2E verdeutlicht dies exemplarisch für eine Messreihe, die beim Eingang des Lebensmittels an der Verkaufsstätte 16 erfolgt. Die Messreihe führt zu einer durchschnittlichen Listerienkonzentration L, die deutlich niedriger ist als die auf Basis der Zwillingsinstanz 300 zu erwarten gewesen wäre.

Aus den bekannten Temperaturdaten des Umgebungsparameters der Zwillingsinstanz 300 lässt sich anhand der gemessenen durchschnittlichen Listerienkonzentration ableiten, dass die initiale Listerienkonzentration $L_0$ vermutlich geringer war, als auf Basis der Zwillingsvorlage 200 angenommen worden war. Dies führt dazu, dass die Wahrscheinlichkeitsverteilung für die Listerienkonzentration $L_0$ in der Zwillingsinstanz angepasst wird, wie die gestrichelte Linie in Fig. 2E verdeutlicht. Die Ermittlung der aktualisierten Wahrscheinlichkeitsverteilung ist dabei wie zuvor genannt mittels des Bayesian-Updating-Verfahrens erfolgt.

Eine Messung der beschriebenen Art, die Auswirkungen auf die Wahrscheinlichkeitsverteilung der initialen Keimbelastung $L_0$ nimmt, kann auch schon unmittelbar nach der Herstellung des Lebensmittels, vorliegend des Schweineschnitzels, erfolgen. In diesem Falle können bei der Übermittlung der Aufforderung vom Rechner 110 zum Zentralserver 100 entsprechende Messdaten mitgesendet werden, die in der Zwillingsinstanz 300 abgelegt werden oder unmittelbar der Anpassung der Wahrscheinlichkeitsverteilungen dienen, beispielsweise auf Basis der Verwendung des Bayesian-Updating-Verfahrens. Neben der Auswirkung auf die Zwillingsinstanz 300 kann eine Messung, insbesondere in Form der genannten umfangreichen Messreihe, auch Einfluss auf die Zwillingsvorlage 200 nehmen. Die gemessenen Daten zur Listerienkonzentration L sowie die bekannte und in der Zwillingsinstanz 300 abgelegte Historie in Hinblick auf die Temperatur seit der Herstellung des Schweineschnitzels gestatten es zusammen mit einer Vielzahl weiterer Messungen an anderen Chargen des gleichen Lebensmittelproduktes aus dergleichen Produktionsstätte, die Wahrscheinlichkeitsverteilungen 208 der Modellparameter anzupassen. Dies erfolgt jedoch vorzugsweise nicht automatisch, sondern unter Sichtung und Anpassung von Fachpersonal.

Die Lebensmittel der ursprünglichen Charge werden in den Verkaufsstätten 16 angeboten. Während die Lebensmittel sich dort in der gekühlten Auslage befinden, ist jederzeit über den jeweiligen digitalen Zwilling eine Prüfung möglich, wie lange sich die Zielvariablen, wie insbesondere die Listerienkonzentration im zulässigen Bereich befinden.

Dies ist insbesondere auch für den Fall vorteilhaft, wenn es durch Störungen zu einer ungeplanten Erwärmung der Lebensmittel kommt. Fällt beispielsweise ein Kühlaggregat für eine Zeit aus, so kann unter Berücksichtigung dessen geprüft werden, welchen Einfluss dies auf die gegenwärtige und die prognostizierte Haltbarkeit nimmt. So kann gegebenenfalls nach einem Ausfall des Kühlaggregats entschieden werden, dass das Lebensmittel nicht mehr verkauft werden kann oder mit einem neuen Haltbarkeitsdatum oder Verbrauchsdatum versehen werden muss, bevor es wieder angeboten werden kann.

Die Lebensmittel können zum Zwecke der Prüfung durch den Kunden beim Eingang in die Verkaufsstätte mit einem Kennzeichen versehen werden, insbesondere der um die Verkaufsstätte ergänzten Chargennummer, die auch im digitalen Zwilling 300 gespeichert ist. Das Kennzeichen kann beispielsweise in Form eines Barcodes oder eines NFC-Tags angebracht werden. Zweckmäßig ist es auch, wenn erst beim Eingang in der Verkaufsstätte das im Lichte der Temperaturdaten aktuelle Haltbarkeitsdatum und/oderVerbrauchsdatum auf dem Lebensmittel in lesbarer Form abgebracht wird.

Die Kunden können auf Basis der Kennzeichnung bei Bedarf mit einem Programm, insbesondere auf ihrem Mobiltelefon 117, das fragliche Lebensmittel scannen und somit auf Daten des digitalen Zwillings 300 oder hieraus abgeleitete Daten zugreifen. Neben der schlichten Abfrage von im digitalen Zwilling gespeicherten Daten, beispielsweise den Temperaturdaten, können die Kunden insbesondere auch die Zielvariablen abfragen. So kann der Kunde insbesondere die Wahrscheinlichkeit ermitteln, mit der die Listerienkonzentration in einem für Kinder und Erwachsene unkritischen Bereich ist. Der Kunde kann darüber hinaus jedoch auch einen strengeren Maßstab anlegen und die Information erlangen, mit welcher Wahrscheinlichkeit die Listerienkonzentration auch in einem für Kleinkinder unkritischen Bereich ist. Eine andere Form der möglichen Datenpräsentation für den Kunden könnte vorsehen, für welche Zielgruppe, beispielsweise Erwachsene, Heranwachsende, Kinder, Kleinkinder, Säuglinge das Lebensmittel noch wie lange mit an Sicherheit grenzender Wahrscheinlichkeit, beispielsweise mit einer Wahrscheinlichkeit von mindestens 99,99%, gesundheitlich unproblematisch ist.

Darüber hinaus ist es dem Kunden auch möglich, eine Prognose zur Zielvariablen zu erhalten, die von prognostizierten zukünftigen Daten zu den Umgebungsparametern, vorliegend also primär der Temperatur, abhängt. So ist es beispielsweise möglich, dass der Kunde über das Programm auf seinem Mobiltelefon 117 eine Prognose abfragt, wie lange die Listerienkonzentration auf dem Lebensmittel noch im unkritischen Bereich verbleibt, wenn er das Lebensmittel bei aktueller Umgebungstemperatur innerhalb von 30 Minuten nach Hause und bis zum Kühlschrank 20 transportiert und ab dann im Kühlschrank bei einer Temperatur von 7°C gelagert wird.

Die Berechnung kann dabei wahlweise durch den Server 100 oder durch die Mobiltelefone 117 erfolgen. Eine Speicherung der Temperaturdaten im digitalen Zwilling auf Basis von für die Zukunft prognostizierten Temperaturdaten findet üblicherweise nicht statt. Fig. 2F verdeutlicht auf der rechten Seite eine mögliche Abfragemaske auf dem Mobiltelefon 117 eines Kunden. Wie bereits beschrieben, kann der Kunde hier prognostizierte Daten zum Umgebungsparameter der Lagertemperatur angeben, die bei der Errechnung der zukünftigen Listerienkonzentration L sowie ggf. anderer Zielvariablen herangezogen werden können. Die Zwillingsinstanz 300 auf der linken Seite in Fig. 2F verdeutlicht diese Berücksichtigung durch den gestrichelt dargestellten prognostizierten Temperaturverlauf.

Wenn der Kunde ein Lebensmittel erwirbt, so trennt sich dessen Weg von den an der Verkaufsstätte 16 verbleibenden Lebensmitteln der gleichen Charge und somit der gleichen Zwillingsinstanz 300. Grundsätzlich ist denkbar, dass hiermit wiederum die Duplizierung der Zwillingsinstanz einhergeht. In der Praxis dürfte dies jedoch üblicherweise zu aufwändigsein, so dass alle weiteren Abfragen von Zielvariablen ab diesem Zeitpunkt vorzugsweise unter Nutzung der Zwillingsinstanz mit dem Zustand beim Eintreffen in derVerkaufsstätte 16 durchgeführt werden.

Wenn stattdessen jedoch eine weitere Duplizierung der Zwillingsinstanz stattfindet, dann könnte es dem Kunden möglich sein, nach dem Kauf des Lebensmittels während der Lagerung des Lebensmittels im Kühlschrank 20 weiterhin, unter Nutzung des Servers 100, Temperaturdaten zum Zwilling300 hinzuzufügen, um weiterhin unter Verwendung tatsächlich konkret gemessener Umgebungsdaten die Qualität des Lebensmittels beurteilen zu können. Sofern der Kunde solche für die Vergangenheit gemessene Umgebungsparameter, insbesondere Temperaturdaten, auf den Zentralserver 100 überträgt, könnten diese also grundsätzlich in einem abgeleiteten digitalen Zwilling für das von ihm erworbene Produkt abgelegt werden. Bevorzugt würde in diesem Fall jedoch eher, dass diese gemessenen Daten auf dem Mobiltelefon 117 selbst abgelegt werden und nur zur Berechnung der aktuellen Eigenschaften temporär und gegebenenfalls wiederholt an den Zentralserver 100 übermittelt werden, so dass dieser unter Nutzung der Zwillingsinstanz 300 beim Eintreffen an der Verkaufsstätte Zielvariablen ermitteln kann. Hierfür muss der Zentralserver 100 die vom Kunden erfassten Daten nicht dauerhaft speichern.

Die in Fig. 2F dargestellt Abfragemaske stellt eine eher komplexe Abfragemaske dar. In der Praxis bietet sich an, dem Endkunden eine einfachere Darstellung zur Verfügung zu stellen, um an der Verkaufsstätte oder später ein Lebensmittel prüfen zu können. Diese könnte sich beispielsweise auf eine Ampeldarstellung oder eine einfache Skala mit einem Frischewert zwischen 0 und 10 beschränken. Zur Umsetzung der ermittelten Zielvariablen auf einen entsprechenden Wert wird üblicherweise eine Bewertungsfunktion verwendet. Wie diese konkret gestaltet ist, sollte auch von der Art der Zielvariablen abhängen. Zielvariablen, die die Konzentration pathogener Bakterien betreffen, sollten sich derart in einer zusammenfassenden Bewertung niederschlagen, dass es bereits ausreicht, wenn eine der Zielvariablen mit relevanter Wahrscheinlichkeit in einem inakzeptablen Bereich ist, um dem Kunden zu signalisieren, dass das Lebensmittel nicht mehr verzehrt werden sollte. Zielvariablen, die Größen betreffen, die gesundheitlich von nachrangiger Bedeutung sind und insbesondere eher Variablen zur Genussqualität darstellen, könnten anders gehandhabt werden. Beispielsweise könnte hier eine Bewertungsfunktion ein Aufaddieren von verschiedenen derartigen Zielvariablen vorsehen, so dass eine eher negative Zielvariable durch eine eher hohe Zielvariable ausgeglichen werden könnte.

Die Verwendung von Bewertungsfunktionen, die auf Basis einer oder mehrerer Zielvariablen einfach erfassbare Ergebnisse liefern, ist nicht auf die ausschließliche Nutzung auf dem Mobiltelefon 117 beschränkt. Daneben können solche Bewertungsfunktionen beispielsweise auch genutzt werden, um an der Vertriebsstätte den Preis des jeweiligen Lebensmittels festzulegen. In dem beschriebenen Beispiel ist die Zwillingsvorlage 200 verwendet worden, die für das Produkt der Schweineschnitzel und die konkrete Produktionsstätte 10 spezifisch ist. Es ist alternativ jedoch auch möglich, dass mehrere alternative Zwillingsvorlagen existieren und/oder eine Zwillingsvorlage mit mehreren alternativen Modellparameter-Sätzen, um weitere Faktoren zu berücksichtigen, wie beispielsweise den Erzeugerbetrieb, von dem das Schwein stammt und/oder die Schlachterei, in der das Schwein geschlachtet und in Schweinhälften zerlegt worden ist.

Verschiedene Zwillingsvorlagen, die für unterschiedliche Lebensmittelprodukte und Produktionsstätten und ggf. auch für weitere Faktoren der Herkunft spezifisch sind, werden auf dem Server vorzugsweise nicht als vollständig voneinander getrennte Zwillingsvorlagen behandelt, sondern sind stattdessen in eine Hierarchie einsortiert. So kann es beispielsweise eine allgemeine Zwillingsvorlage für Schweinefleischprodukte geben, die Grundlage für verschiedene Zwillingsvorlagen unterschiedlicher Schweinefleischprodukte ist. Diese Zwillingsvorlagen für unterschiedliche Schweinefleischprodukte können dann wiederum Grundlage für die für die Produktionsstätte spezifischen Zwillingsvorlagen sein, die ihrerseits Verwendung finden, um in oben beschriebener Weise zur Ableitung der Zwillingsinstanzen genutzt zu werden.

Eine solche Hierarchie gestattet es beispielsweise, fundamentale mathematische Modelle verschiedener Zielvariablen höheren Hierarchiestufen zuzuordnen und diese hieraus einheitlich in niedrigere Hierarchiestufen zu übernehmen, während Wahrscheinlichkeitsverteilungen der jeweiligen Modellparameter den niedrigeren Hierarchiestufen zugeordnet sind.

**Patentansprüche**

1. Verfahren zur Ermittlung von Eigenschaften von Lebensmitteln mit den folgenden Merkmalen:

   a. es wird ausgehend von einer digitalen Zwillingsvorlage (200) eine digitale Zwillingsinstanz (300) als Repräsentation des Lebensmittels erzeugt, und
   b. der digitalen Zwillingsinstanz (300) sind mindestens die folgenden Informationen zugeordnet:

      - für mindestens eine erste Zielvariable (304), die der Beschreibung einer Eigenschaft des Lebensmittels dient, ein mathematisches Modell (306), welches über mindestens einen Modellparameter und mindestens über einen Umgebungsparameter (310) verfügt, und
      - eine Wahrscheinlichkeitsverteilung (308) in Hinblick auf den mindestens einen Modellparameter des mathematischen Modells (306) der ersten Zielvariable, und

   c. im Zuge der Handhabung des Lebensmittels bis zum Erreichen einer Verkaufsstätte (16) und/oder an der Verkaufsstätte (16) erfolgt mindestens eine Messung des mindestens einen Umgebungsparameters (310), wobei die hierbei ermittelten Werte des Umgebungsparameters (310) in der Zwillingsinstanz (300) zugeordneter Weise abgespeichert werden, und
   d. unter Verwendung des mathematischen Modells (306) der ersten Zielvariable (304), der Wahrscheinlichkeitsverteilung (308) des mindestens einen Modellparameters und der ermittelten Werte des mindestens einen Umgebungsparameters (310) wird für den aktuellen Zeitpunkt oder einen zukünftigen Zeitpunkt eine Wahrscheinlichkeitsverteilung (320) in Hinblick auf die mindestens eine Zielvariable (304) ermittelt.

2. Verfahren nach Anspruch 1 mit mindestens einem der folgenden weiteren Merkmale:

   a. unter Verwendung der Wahrscheinlichkeitsverteilung (320) in Hinblick auf die mindestens eine Zielvariable (304) wird durch Aufsummieren der Fläche unter der Wahrscheinlichkeitsverteilung (320) ermittelt, mit welcher kumulierten Wahrscheinlichkeit die Zielvariable (304) des Lebensmittels unterhalb bzw. oberhalb eines vorgegebenen Grenzwertes für die Zielvariable liegt, und/oder
   b. unter Verwendung der Wahrscheinlichkeitsverteilung (320) in Hinblick auf die mindestens eine Zielvariable (304) wird durch Aufsummieren der Fläche unter der Wahrscheinlichkeitsverteilung (320) ermittelt, welcher Wert der Zielvariable (304) statistisch bei einem vorgegebenen Anteil des Lebensmittels unterschritten bzw. überschritten ist.

3. Verfahren nach einem der vorstehenden Ansprüche mit mindestens einem derfolgenden Merkmale:

   a. das mathematische Modell (306) der digitalen Zwillingsinstanz (300) mindestens einer Zielvariable (304) ist ein mathematisches Modell (306) zur Ermittlung einer der folgenden mikrobiologischen Zielvariablen:

      - Konzentration in Hinblick auf beliebige Keime und/oder
      - Konzentration von Listerien und/oder
      - Konzentration von Lactobacillales und/oder
      - Konzentration von Cronobacter und/oder
      - Konzentration von Bacillus cereus und/oder

- Konzentration von Campylobacter und/oder
- Konzentration von Salmonellen und/oder
- Konzentration von Shigellen und/oder
- Konzentration von Staphylococcus aureus und/oder
- Konzentration von Pseudomonas spp. und/oder
- Konzentration von Schimmelpilz und/oder
- Konzentration von Aspergillus spp., und/oder

b. das mathematische Modell (306) der digitalen Zwillingsinstanz (300) mindestens einer Zielvariable (304) ist ein mathematisches Modell (306) zur Ermittlung einer der folgenden biochemischen Zielvariablen:

- Bräunungsgrad und/oder
- Reifegrad und/oder
- Säuregehalt und/oder
- Zuckergehalt und/oder
- Konzentration von Vitamine und/oder
- Konzentration oxidierter Fette, und/oder

c. das mathematische Modell (306) der digitalen Zwillingsinstanz (300) mindestens einer Zielvariable (304) ist ein mathematisches Modell (306) zur Ermittlung einer der folgenden physikalischen Zielvariablen:

- Farbe und/oder
- Textur und/oder
- Wassergehalt und/oder
- Druckfestigkeit und/oder
- Trockenmasse, und/oder

d. das mathematische Modell (306) der digitalen Zwillingsinstanz (300) mindestens einer Zielvariable (304) ist ein mathematisches Modell (306) zur Ermittlung einer der folgenden subjektiven oder aggregierten Zielvariablen:

- Geschmack und/oder
- Frische und/oder
- Qualität.

4. Verfahren nach einem der vorstehenden Ansprüche mit dem folgenden Merkmal:

a. die Umgebungsparameter (310), die in der Zwillingsinstanz (300) zugeordneter Weise abgespeichert werden, umfassen mindestens einen der folgenden Umgebungsparameter (310):

- Temperatur des Lebensmittels und/oder Umgebungstemperatur im Raum, in dem das Lebensmittel gelagert ist, und/oder
- Umgebungsluftfeuchtigkeit im Raum, in dem das Lebensmittel gelagert ist, und/oder
- Zusammensetzung von Luft, die das Produkt umgibt, wobei der Umgebungsparameter vorzugsweise die Konzentration mindestens eines Spurengasen ($CO_2$, Ethylen) ist.

5. Verfahren nach einem der vorstehenden Ansprüche mit dem folgenden Merkmal:

a. die digitale Zwillingsinstanz (300) als Repräsentation des Lebensmittels wird im Zuge der Produktion in einem Fertigungsbetrieb erzeugt, insbesondere

- bei Fleischprodukten im Zerlegebetrieb, oder
- bei Fischereiprodukten während des Fangs,

vorzugsweise mit dem zusätzlichen Merkmal:
b. die Modellparameter der digitalen Zwillingsinstanz (300) des Lebensmittels sind bis mindestens zum Zeitpunkt, zu dem das Lebensmittel den Fertigungsbetrieb verlässt, gegenüber der digitalen Zwillingsvorlage (200) unverändert.

**6.** Verfahren nach einem der vorstehenden Ansprüche mit dem folgenden Merkmal:

a. die digitale Zwillingsinstanz (300) als Repräsentation des Lebensmittels wird im Zuge einer Warenannahme und/oder eines Gefahrenübergangs einer Transportladung von Lebensmitteln erzeugt, vorzugsweise mit dem zusätzlichen Merkmal:
b. im Zuge der Warenannahme bzw. des Gefahrenübergangs des Lebensmittels erfolgt mindestens eine Messung einer Zielvariablen (304) an mindestens einem Einzel-Lebensmittel und die Erzeugung der digitalen Zwillingsinstanz (300) erfolgt derart, dass mindestens eine Wahrscheinlichkeitsverteilung (308) mindestens eines Modellparameters des mathematischen Modells (306) der Zielvariable in Abhängigkeit des Messergebnisses in der digitalen Zwillingsinstanz (300) hinterlegt wird.

**7.** Verfahren nach einem der vorstehenden Ansprüche mit den folgenden Merkmalen:

a. im Zuge der Handhabung des Lebensmittels bis zum Erreichen einer Verkaufsstätte (16) und/oder an der Verkaufsstätte (16) erfolgt mindestens eine Messung einer Zielvariablen (304) der Zwillingsinstanz des Lebensmittels, und
b. in Abhängigkeit des Ergebnisses dieser Messung erfolgt eine Aktualisierung der Zwillingsinstanz (300), insbesondere eine Aktualisierung der Wahrscheinlichkeitsverteilung (308) mindestens eines Modellparameters des mathematischen Modells (306) der Zielvariable.

**8.** Verfahren nach Anspruch 7 mit dem folgenden Merkmal:

a. in Abhängigkeit des Ergebnisses der Messung erfolgt eine Aktualisierung der Wahrscheinlichkeitsverteilung (308) des Modellparameters betreffend eines Initialwertes ($L_0$) der Zielvariablen (304) bei Herstellung des Lebensmittels.

**9.** Verfahren nach Anspruch 7 oder 8 mit dem folgenden Merkmal:

a. bei Aktualisierung des mindestens einen Modellparameters des mathematischen Modells (306) der Zielvariable (304) wird die zuvor geltende Wahrscheinlichkeitsverteilung (308) des Modellparameters zum Zwecke der späteren Nachvollziehbarkeit in einem Speicher der Zwillingsinstanz (300) belassen.

**10.** Verfahren nach einem der vorstehenden Ansprüche mit den folgenden Merkmalen:

a. im Zuge der Handhabung des Lebensmittels bis zum Erreichen einer Verkaufsstätte (16) und/oder an der Verkaufsstätte (16) erfolgt mindestens eine Messung einer Zielvariablen (304) der Zwillingsinstanz (300) des Lebensmittels, und
b. auf Basis der Ergebnisse dieser Messung sowie einer Mehrzahl weiterer Messungen von Lebensmitteln, deren Zwillingsinstanzen (300) von dergleichen Zwillingsvorlage (200) abgeleitet wurde, erfolgt eine Aktualisierung der Zwillingsvorlage (200).

**11.** Verfahren nach einem der vorstehenden Ansprüche mit den folgenden Merkmalen:

a. im Zuge der Handhabung des Lebensmittels bis zum Erreichen einer Verkaufsstätte (16) und/oder an der Verkaufsstätte (16) erfolgt mindestens eine Messung einer Zielvariablen (304) der Zwillingsinstanz (300) des Lebensmittels, und
b. sofern die Messung einen gesundheitlich bedenklichen Wert der Zielvariable (304) ergibt, der bezogen auf die auf Basis der Zwillingsinstanz (300) ermittelte Wahrscheinlichkeit der Zielvariable (304) unwahrscheinlich ist, erfolgt, gegebenenfalls abhängig und/oder differenziert nach der Schwere der gesundheitlichen Bedenken, mindestens eines der folgenden Maßnahmen:

- Anpassung anderer Zwillingsinstanzen (300) anderer Lebensmittel, insbesondere anderer Lebensmittel, die der gleichen Charge wie der des gemessenen Lebensmittels entstammen, und/oder
- Erzeugung einer Warnmitteilung, insbesondere einer Warnmitteilung, die Zwillingsinstanzen anderer Lebensmittel zugeordnet wird, insbesondere anderer Lebensmittel, die der gleichen Charge wie der des gemessenen Lebensmittels entstammen.

**12.** Verfahren nach einem der vorstehenden Ansprüche mit dem folgenden Merkmal:

a. die Ermittlung der Wahrscheinlichkeit in Hinblickaufdie mindestens eineZielvariable (304) unter Verwendung der Zwillingsinstanz (300) erfolgt nach Auslösung über ein Abfragegerät (117) eines Kunden oder Verbrauchers an der Verkaufsstätte oder nach Erwerb des Lebensmittels,

gegebenenfalls mit mindestens einem der folgenden zusätzlichen Merkmale:

b. das Abfragegerät (117) ist ein Mobilfunkgerät (117), und/oder

c. auf dem Abfragegerät (117) des Kunden wird angezeigt, ob eine oder mehrere Zielvariablen (304) sich mit vorgegebener Wahrscheinlichkeit in einem gesundheitlich unbedenklichen Bereich befinden, und/oder

d. auf dem Abfragegerät (117) des Kunden wird angezeigt, mit welcher Wahrscheinlichkeit sich eine oder mehrere Zielvariablen (304) in einem gesundheitlich unbedenklichen Bereich befinden, und/oder

e. die Ermittlung der Wahrscheinlichkeit in Hinblick auf die mindestens eine Zielvariable erfolgt unter Einbeziehung von prognostizierten oder gemessenen Daten zum mindestens einen Umgebungsparameter (310) derZwillingsinstanz (300), wobei hierfürdurch den Kunden oder Verbraucher insbesondere Daten zur Verfügung gestellt werden, die widergeben, unter welchen Bedingungen gelagert wurde oder werden wird, und/oder Daten zur Verfügung gestellt werden, die widergeben, wie lange und/oder unter welchen Bedingungen das Lebensmittel transportiert wurde oder werden wird, bis es eine Kühleinrichtung (120) des Kunden oder Verbrauchers erreicht, wobei diese Daten vorzugsweise zumindest teilweise im Abfragegerät hinterlegt sind, und/oder

f. im Falle, dass eine Warnmitteilung der Zwillingsinstanz (300) zugeordnet ist, wird die auf den Abfragegerät (117) ausgegeben.

13. Computerprogrammprodukt oder Computersystem mit dem folgenden Merkmal:

Das Computerprogrammprodukt umfasst Befehle bzw. das Computersystem umfasst ein Computerprogrammprodukt mit Befehlen, die bei der Ausführung des Programms durch einen Computer diesen veranlasst, das Verfahren nach einem der vorstehenden Ansprüche durchzuführen.

**Fig. 1**

$$\frac{dL}{dt} = \alpha_0 T(t) \left( L - L_0 e^{-\lambda_0 \int T(t')dt'} \right)$$

**Fig. 2A**

**Fig. 2B**

**Fig. 2C**

*Fig. 2D*

302

306

304 $L$ | $\frac{dL}{dt} = \alpha_0 T(t)\left(L - L_0 e^{-\lambda_0 \int T(t')dt'}\right)$

ID: 123456789-A

308

300

$x$

$y$

310 $T(t)$ $t$

## Fig. 2E

302

306

304 $L$ | $\frac{dL}{dt} = \alpha_0 T(t)\left(L - L_0 e^{-\lambda_0 \int T(t')dt'}\right)$

ID: 123456789-A

308

300

$x$

$y$

310 $T(t)$ $t$

## Fig. 2F

| ID: | 123456789-A |
| --- | --- |
| Lebensmittel/ Food: | Schweineschnitzel/ Pork Cutlet |
| Sicherheitslevel/ Security Level | 99,99% ▼ |

Transportdaten/Transportation Data:

| 30 min ▼ | 20°C ▼ |
| --- | --- |

Heimische Lagerung/Domestic Storage

7°C ▼

Unbedenklicher Verzehr möglich bis/
Safe Consumption until

| Erwachsene/Adults | Feb 19, 2021 |
| --- | --- |
| Kinder/Children | Feb 17, 2021 |
| Säuglinge/Babies | Feb 14, 2021 |

117

*Fig. 3A*

*Fig. 3B*

*Fig. 3C*

*Fig. 3D*

Fig. 3E

Fig. 3F

Europäisches
Patentamt

European
Patent Office

Office européen
des brevets

**EUROPÄISCHER RECHERCHENBERICHT**

Nummer der Anmeldung

EP 20 18 9656

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Bwcon: "Revolution in der Lieferkette: FreshIndex soll Haltbarkeit von Lebensmitteln in Echtzeit berechnen", INTERNET ARTICLE, 21. März 2018 (2018-03-21), XP055763631, Gefunden im Internet: URL:https://www.bwcon.de/fileadmin/bwcon/bwcon/Presse/Pressemitteilungen/21.03.2018_PM_FreshIndex.pdf [gefunden am 2021-01-11] * Seite 1 - Seite 2 *<br>----- | 1-13 | INV. G06Q10/08 G16Y20/10 |
| X | Frank Meßing: "Test bei Metro: Handy zeigt Frischegrad von Lebensmitteln an", INTERNET ARTICLE, 23. August 2019 (2019-08-23), XP055764844, Gefunden im Internet: URL:https://www.ikz-online.de/wirtschaft/wirtschaft-in-nrw/test-bei-metro-handy-zeigt-frischegrad-von-lebensmitteln-an-id226857053.html [gefunden am 2021-01-13] * Seite 1 - Seite 3 *<br>----- | 1-13 | |
| X | Timm Seckel: "Die neue App der Metro soll das Haltbarkeitsdatum überflüssig machen", INTERNET ARTICLE, 19. August 2019 (2019-08-19), XP055764847, Gefunden im Internet: URL:https://www.handelsblatt.com/unternehmen/handel-konsumgueter/lebensmittelverschwendung-die-neue-app-der-metro-soll-das-haltbarkeitsdatum-ueberfluessig-machen/24912656.html?ticket=ST-3371972-hjcrOaA2OkMhIJYi9uqR-ap1 [gefunden am 2021-01-13] * Seite 1 - Seite 4 *<br>-----<br>-/-- | 1-13 | RECHERCHIERTE SACHGEBIETE (IPC)<br><br>G06Q G16Y |

Der vorliegende Recherchenbericht wurde für alle Patentansprüche erstellt

| Recherchenort | Abschlußdatum der Recherche | Prüfer |
|---|---|---|
| München | 14. Januar 2021 | Krafft, Gerald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

................................................................
& : Mitglied der gleichen Patentfamilie, übereinstimmendes Dokument

EPO FORM 1503 03.82 (P04C03)

Seite 1 von 3

Europäisches Patentamt
European Patent Office
Office européen des brevets

# EUROPÄISCHER RECHERCHENBERICHT

Nummer der Anmeldung

EP 20 18 9656

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EVA WERA RITTER ET AL: "The Fresh Index: A Real-Time Shelf Life Indicator", PROCEEDINGS OF THE 13THINTERNATIONAL EUROPEAN FORUM ON SYSTEM DYNAMICS AND INNOVATION IN FOOD NETWORKS, ORGANIZED BY THE INTERNATIONAL CENTER FOR FOOD CHAIN AND NETWORK RESEARCH, UNIVERSITY OF BONN, GERMANY FEBRUARY 18-22, 2019, GARMISCH-PARTENKIRCHE, 22. Februar 2019 (2019-02-22), XP055763649, DOI: 10.18461/pfsd.2019.1902 * Seite 15 - Seite 16 * ----- | 1-13 | |
| X | Fi-Impact: "tsenso FIWARE Case Study tsenso Case Study", INTERNET ARTICLE, 31. März 2016 (2016-03-31), XP055763619, Gefunden im Internet: URL:http://www.fi-impact.eu/media/FI-IMPACT_FInish_Tsenso_FIWARECaseStudy_Final_3103 16.pdf [gefunden am 2021-01-11] * Seite 1 - Seite 4 * ----- | 1-13 | |
| X | Michael A. Bourlakis ET AL: "Intelligent Agrifood Chains and Networks", 6. Mai 2011 (2011-05-06), Wiley-Blackwell, XP055519826, ISBN: 978-1-4051-8299-7 Seiten ToC,Ch03-Ch07,Ch09,, * Kapitel 3 bis 6 * ----- | 1-13 | RECHERCHIERTE SACHGEBIETE (IPC) |
| X | US 2018/120169 A1 (JACKSON MARTIN KIRK [GB] ET AL) 3. Mai 2018 (2018-05-03) * Absatz [0016] - Absatz [0064]; Abbildungen 1,2 * ----- -/-- | 1-13 | |

Der vorliegende Recherchenbericht wurde für alle Patentansprüche erstellt

| Recherchenort | Abschlußdatum der Recherche | Prüfer |
|---|---|---|
| München | 14. Januar 2021 | Krafft, Gerald |

Europäisches
Patentamt

European
Patent Office

Office européen
des brevets

# EUROPÄISCHER RECHERCHENBERICHT

**Nummer der Anmeldung**

EP 20 18 9656

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | Deloitte: "IoT powered by Blockchain - How Blockchains facilitate the application of digital twins in IoT", INTERNET ARTICLE, 31. Mai 2018 (2018-05-31), XP055763908, Gefunden im Internet: URL:https://www2.deloitte.com/content/dam/Deloitte/de/Documents/Innovation/IoT-powered-by-Blockchain-Deloitte.pdf [gefunden am 2021-01-12] * Seiten 8,16 *  ----- | 1-13 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 14. Januar 2021 | Krafft, Gerald |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument
.........................................................................
& : Mitglied der gleichen Patentfamilie, übereinstimmendes
Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 18 9656

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-01-2021

| Im Recherchenbericht angeführtes Patentdokument | Datum der Veröffentlichung | Mitglied(er) der Patentfamilie | Datum der Veröffentlichung |
|---|---|---|---|
| US 2018120169 A1 | 03-05-2018 | CN 107743631 A | 27-02-2018 |
| | | EP 3281156 A1 | 14-02-2018 |
| | | GB 2537170 A | 12-10-2016 |
| | | JP 2018518782 A | 12-07-2018 |
| | | US 2018120169 A1 | 03-05-2018 |
| | | WO 2016162545 A1 | 13-10-2016 |

Für nähere Einzelheiten zu diesem Anhang : siehe Amtsblatt des Europäischen Patentamts, Nr.12/82

EPO FORM P0461

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 60217329 T2